# EUROPÄISCHE PATENTANMELDUNG

(11) **EP 1 775 584 A2**
(43) Veröffentlichungstag der Anmeldung: **18.04.2007**
(21) Anmeldenummer: 06119757.0
(22) Anmeldetag: 30.08.2006
(51) Int. Cl.: G01N 33/28

(54) **Verfahren und Vorrichtung zur Erkennung der Qualität eines Kraftstoffs für Brennkraftmaschine**

(30) Priorität: 11.10.2005 DE 102005048706
(71) Anmelder: SIEMENS AKTIENGESELLSCHAFT, 80333 München (DE)
(72) Erfinder: Kettl, Thomas, 94348 Atting (DE); Zhang, Hong, 93105 Tegernheim (DE)

(57) **Zusammenfassung**

Die Erfindung betrifft ein Verfahren sowie eine Vorrichtung zur Beurteilung der Qualität des Kraftstoffs, der in einer Brennkraftmaschine (1) momentan verbrannt wird. Heute verwendete Kraftstoffe können eine unterschiedliche Dichte beziehungsweise unterschiedliche Heizwerte aufweisen. Auch beim Auffüllen des Kraftstofftanks (7) kann sich im Kraftstofftank (7) die Qualität des Kraftstoffs ändern, da verschiedene Kraftstoffe vermischt werden. Beispielsweise kann eine geringe Dichte beziehungsweise ein geringer Heizwert zu einer verringerten Leistung der Brennkraftmaschine (1) führen. Erfindungsgemäß wird daher vorgeschlagen, mit Hilfe eines Sensors (2,2a), mit dem beispielsweise den Verlauf des Brennraumdrucks und/oder einen Lambdawert erfasst wird, und mit Hilfe eines Algorithmus einen kraftstoffspezifischen Faktor k zu ermitteln. In weiterer Ausgestaltung der Erfindung werden mit Hilfe des Faktors k die Betriebsparameter (Einspritzmenge, Einspritzbeginn, Einspritzende, Einspritzmuster, Abgasrückführrate usw.) korrigiert.

## Beschreibung

Die Erfindung betrifft ein Verfahren bzw. eine Vorrichtung zur Beurteilung der Qualität eines Kraftstoffs, insbesondere eines Diesel- oder Benzinkraftstoffs nach der Gattung der nebengeordneten Ansprüche 1 und 9. Es ist bereits bekannt, dass Kraftstoffe für Verbrennungsmotoren, insbesondere Dieselkraftstoffe eine sehr unterschiedliche Dichte bzw. sehr unterschiedliche Heizwerte aufweisen können. Dieselkraftstoffe werden in der Regel mit Additiven vermischt, die insbesondere die Zündwilligkeit, Gleit- und Schmiereigenschaften sowie die Abgasemissionen beeinflussen können. Ein Maß für die Qualität der Zündwilligkeit von Dieselkraftstoffen ist beispielsweise die Cetanzahl CZ, die an einem besonders konstruierten EinZylinder-Prüfmotor ermittelt wird. An diesem Prüfmotor können Vergleichskraftstoffe getestet werden, die mit dem sehr zündwilligen n-Cetan C₁₆H₃₄ und dem zündträgen α-Methylnaphtalin C₁₁H₁₀ verglichen werden. Die Cetanzahl wird in einem Bereich zwischen 0 und 100 festgelegt. Eine zu niedrige Cetanzahl belastet die Umwelt, besonders durch Rußbildung bei Dieselmotoren. Üblicherweise liegt in Deutschland die Cetanzahl CZ zwischen 50 und 55.

Die Cetanzahl von Dieselkraftstoffen kann sehr unterschiedlich sein, insbesondere ist sie bei Bio-Diesel verhältnismäßig niedrig. Ein weiteres Problem besteht darin, dass beim Nachtanken die im Kraftstofftank befindlichen Kraftstoffe mit unterschiedlichen Qualitäten vermischt werden. Dadurch ist es für das Motorsteuergerät sehr schwierig, beispielsweise bei einem Common-Rail-Einspritzsystem eine einzuspritzende Kraftstoffmenge so zu steuern, dass eine optimale Verbrennung insbesondere mit minimalen Abgasemissionen entsteht. Hinzu kommt, dass bei einem Kraftstoff mit einer geringeren Dichte die der Brennkraftmaschine zugeführte Energie geringer ist als bei einem Kraftstoff mit einer höheren Dichte. Dadurch wird auch die Motorleistung in unerwünschtem Maße beeinflusst. Alle diese Faktoren sowie die aktuellen Betriebsparameter müssen von einem Motorsteuergerät so berücksichtigt werden, dass bei einer entsprechenden Drehmomentenanforderung eine optimale einzuspritzende Kraftstoffmenge berechnet werden kann. Bei bekannten Motorsteuergeräten werden diese Anforderungen jedoch nur unzureichend erfüllt.

Bisher wurde dieses Problem insbesondere bei Dieselkraftstoffen dadurch gelöst, dass in dem Kraftstofftank ein optischer Sensor eingebaut wurde. Der optische Sensor hat dabei die Aufgabe, aufgrund der Trübung des Kraftstoffes auf die Qualität zu schließen. Dieses Verfahren arbeitet jedoch relativ unzuverlässig, da einzelne Kraftstoffhersteller dem Kraftstoff einen Farbstoff beimischen. Dadurch ist eine objektive Beurteilung des verwendeten Kraftstoffs erheblich erschwert.

Der Erfindung liegt die Aufgabe zugrunde, die Qualität eines Kraftstoffs, der sich im Kraftstofftank eines Kraftfahrzeugs befindet, direkt während des Betriebes des Kraftfahrzeugs zu beurteilen. Diese Aufgabe wird mit den kennzeichnenden Merkmalen der nebengeordneten Ansprüche 1 und 9 gelöst.

Bei dem erfindungsgemäßen Verfahren bzw. der Vorrichtung zur Beurteilung der Qualität eines Kraftstoffs mit den kennzeichnenden Merkmalen der nebengeordneten Ansprüche 1 und 9 ergibt sich der Vorteil, dass die Qualität des Kraftstoffs, der sich aktuell im Kraftstofftank eines Kraftfahrzeugs befindet, mit relativ großer Sicherheit und objektiv beurteilt werden kann. Das kann beispielsweise mit einem Sensor erreicht werden, der den während der Verbrennung des Kraftstoffs entstehenden Druckverlauf im Brennraum der Brennkraftmaschine erfasst und mit einem vorgegebenen Algorithmus auswertet. Eine vorteilhafte alternative Lösung wird darin gesehen, dass der von einer Lambda-Sonde gemessene Lambdawert ausgewertet wird. Der Lambdawert wird dabei bei einem konstanten Betriebspunkt der Brennkraftmaschine gemessen und mit gespeicherten Sollwerten verglichen. Je nach Kraftstoffqualität kann sich bei konstantem Betriebspunkt ein unterschiedlicher Lambdawert ergeben, da verschiedene Kraftstoffe eine unterschiedliche Dichte bzw. einen unterschiedlichen Heizwert aufweisen können und sich dadurch bei der Verbrennung im Brennraum der Brennkraftmaschine ein unterschiedliches Verbrennungsbild ergeben kann. Als besonders vorteilhaft wird dabei angesehen, dass die verschiedenen Kraftstoff-Typen bzw. -Qualitäten mit einem einfachen Faktor bewertet werden können, so dass dadurch ein Vergleich der Kraftstoff-Qualitäten sehr einfach durchgeführt werden kann.

Durch die in den abhängigen Ansprüchen aufgeführten Maßnahmen sind vorteilhafte Weiterbildungen und Verbesserungen des im Anspruch 1 angegebenen Verfahrens bzw. der im Anspruch 9 vorgegebenen Vorrichtung gegeben. Eine besonders vorteilhafte Möglichkeit der Weiterbildung wird darin gesehen, dass der Faktor einem Motorsteuergerät zugeführt wird. Das Motorsteuergerät, das bei einer modernen Brennkraftmaschine bereits schon vorhanden ist, kann dann mit Hilfe des Faktors und weiterer Betriebsparameter, beispielsweise der Steuerung des Einspritzzeitpunktes, Steuerung der Einspritzdauer, der Abgasrückführung, Änderung der Mehrfacheinspritzung, Rate shaping usw. die Einspritzmenge optimal bestimmen und dabei einen Kraftstoffinjektor auf direktem oder indirektem Wege entsprechend steuern. Beispielsweise kann der Einspritzbeginn und/oder der Einspritzdruck verändert und dadurch die Emissionsziele zu erreichen, wenn eine weniger gute Kraftstoff-Qualität festgestellt wurde. Außerdem kann die einzuspritzende Kraftstoffmenge reduziert werden, wenn die Kraftstoffqualität überdurchschnittlich hoch ist.

Ein besonders einfaches Verfahren zur Ermittlung der Kraftstoff-Qualität besteht darin, wenn der während der Verbrennung innerhalb eines vorgegebenen Winkelbereiches der Kurbelwelle auftretende Druckverlauf im Brennraum der Brennkraftmaschine aufgezeichnet und ausgewertet wird. Beispielsweise kann über die Druckkurve oder eine dem Druckverlauf entsprechende Kurve das Integral gebildet werden, so dass daraus die gewonnene Arbeit bzw. gewonnene Energiemenge bestimmt werden kann. Durch Vergleich der ermittelten Werte mit gespeicherten oder gerechneten Werten ist somit eine leichte Beurteilung der Kraftstoffqualität möglich, um Rückschlüsse auf den Heizwert beziehungsweise die Dichte des eingespritzten Kraftstoffs zu erhalten.

Eine weitere vorteilhafte Möglichkeit zur Beurteilung der Qualität des Kraftstoffs wird auch in der Ermittlung des Zündverzugs gesehen. Der Zündverzug ergibt sich aus einem verzögerten Anstieg des Brennraumdrucks. Da verschiedene Kraftstoffqualitäten im Brennraum auch unterschiedlich verschobene Druckkurven erzeugen, ergibt sich aus der Verschiebung der Druckkurve, die ein objektives Maß für den Zündverzug ist, eine sehr einfache und vorteilhafte Möglichkeit zur Bestimmung der Kraftstoffqualität.

Durch Bewertung des Zündverzuges kann ebenfalls auf die Bewertung des Kraftstoffes, insbesondere bei einem Dieselkraftstoff geschlossen werden, da der Zeitpunkt des Druckanstiegs ein Maß für die Zündwilligkeit des Dieselkraftstoffs ist. Die Zündwilligkeit des Kraftstoffs wird durch die sog. Cetanzahl bestimmt. Je geringer der Zündverzug ist, d.h. je zündwilliger der eingespritzte Kraftstoff ist, umso größer ist die Cetanzahl. Entsprechendes gilt im umgekehrten Fall für eine niedrige Cetanzahl.

Eine weitere vorteilhafte Möglichkeit zur Beurteilung der Qualität des Kraftstoffs besteht auch darin, dass für einen konstanten Betriebspunkt der Brennkraftmaschine ein aktueller Lambdawert gemessen und ausgewertet wird. Der Lambdawert spiegelt das Verhältnis von angesaugter Luftmasse zu eingespritzter Kraftstoffmenge wieder. Der Lambdawert wird üblicherweise mit einer Lambda-Sonde gemessen, die im Abgassystem der Brennkraftmaschine eingebaut ist und den im Abgasstrom enthaltenen Restsauerstoff erfasst. Werden für einen vorgegebenen, konstanten Betriebspunkt der Brennkraftmaschine bei verschiedenen Kraftstoffen unterschiedliche Lambdawerte gemessen, dann kann daraus auf unterschiedliche Kraftstoff-Qualitäten geschlossen werden.

Besonders zuverlässig kann eine Kraftstoff-Qualität bestimmt werden, wenn der Lambdawert für einen vorgegebenen, konstanten Betriebspunkt gemessenen und dann mit einem entsprechenden, gespeicherten Lambda-Sollwert zu vergleichen. Durch den Vergleich erhält man ein Maß für die Beurteilung der Qualität des Kraftstoffes, der momentan in den Brennraum der Brennkraftmaschine eingespritzt wird.

Ein Lambdawert eins bedeutet, dass das Verhältnis aus der angesaugten Luftmasse zu der eingespritzten Kraftstoffmenge den Wert 14,6 erreicht. Bei diesem Verhältnis liefert die Lambda-Sonde den genauesten Messwert, so dass vorzugsweise der Betriebspunkt für die Brennkraftmaschine während der Messung so gewählt wird, dass der gespeicherte Lambda-Sollwert in der Nähe vom Wert eins liegt.

Eine Ermittlung und Beurteilung eines Kraftstoffs wird in vorteilhafter Weise möglichst dann durchgeführt, wenn der Kraftstofftank ganz oder teilweise neu befüllt wird. Denn durch die Vermischung mit dem noch im Kraftstofftank befindlichen restlichen Kraftstoff ergibt sich eine Durchschnittqualität für den Kraftstoff, die für die Verarbeitung im Motorsteuergerät maßgebend ist. Erfindungsgemäß ist daher vorgesehen, wenigstens ein Mal nach dem Auffüllen des Kraftstofftanks die Bewertung der Kraftstoff-Qualität durchzuführen.

Ein Ausführungsbeispiel der Erfindung ist in der schematischen Zeichnung dargestellt und wird in der nachfolgenden Beschreibung näher erläutert.
- Figur 1: zeigt ein Diagramm mit zwei Kraftstoffkurven.
- Figur 2: zeigt ein Blockschaltbild mit einer erfindungsgemäßen Vorrichtung und
- Figur 3: zeigt ein Flussdiagramm, das zur Beurteilung der Kraftstoff-Qualität verwendet werden kann.

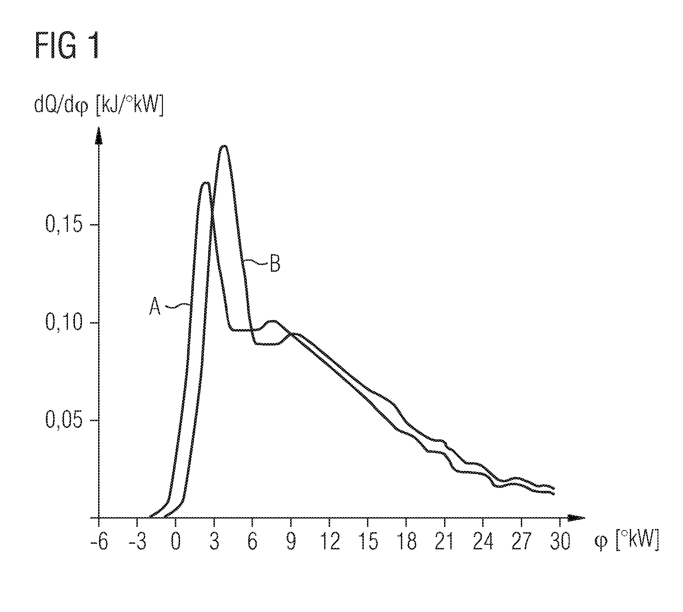

In Figur 1 ist ein Diagramm dargestellt, bei dem zwei unterschiedliche Kraftstoffsorten A und B miteinander verglichen werden. Die beiden Kraftstoffsorten A, B unterscheiden sich in einer unterschiedlichen Cetanzahl CZ. Bei der Kurve A ist die Cetanzahl CZ um etwa zwei Einheiten größer als bei der Kurve B. Auf der Y-Achse des Diagramms ist die spezifische Energiezufuhr dQ/dϕ [kJ/⁰KW] dargestellt (dQ/dϕ ist die Energieänderung pro Kurbelwellenwinkel und °KW bedeutet Kurbelwellenwinkel in Grad). Auf der X-Achse ist der Kurbelwellenwinkel ϕ in Grad angegeben. Die relative Energiezufuhr entspricht dem durch die Verbrennung des Kraftstoffs entstehenden Druckanstieg im Brennraum der Brennkraftmaschine, wenn nach der Kraftstoffeinspritzung die Kurbelwelle ihren oberen Totpunkt entsprechend dem Kurbelwellenwinkel ϕ = 0° überschritten hat. Wie dem Diagramm weiter entnehmbar ist, ist der Anstieg der Kurve A im Bereich 0 - 3° bis zu seinem Maximum sehr steil. Nach Beendigung der Verbrennung und mit größer werdendem Kurbelwellenwinkel fällt dann der Druck im Brennraum relativ rasch ab, weil sich dann das Gasgemisch im Brennraum schnell abkühlt und sich der Kolben wieder nach unten bewegt. Der steile Anstieg der Kurve A bedeutet, dass in diesem Bereich die Zündung des eingespritzten Kraftstoffes erfolgt und dadurch der Brennraumdruck sehr rasch ansteigt.

Die Kurve B weist im Wesentlichen den gleichen Verlauf wie die Kurve A auf, sie ist jedoch etwas nach rechts verschoben. Des Weiteren unterscheidet sich die Kurve B von der Kurve A dadurch, dass die Kurve B eine größere Amplitude aufweist. Wird über die beiden Kurven A beziehungsweise B jeweils das Integral gebildet, dann erhält man die zugeführte Energie, die dem eingespritzten Kraftstoff in Form eines Drehmomentes entnommen werden kann. Die beiden Kurven A,B wurden unter gleichen, konstanten Betriebsbedingungen und gleichen Einspritzmengen der beiden Kraftstoffe A und B ermittelt und wurden bereits im Diagramm der Figur 1 in einer auswertbaren Form dargestellt. Der tatsächliche Druckverlauf im Brennraum der Brennkraftmaschine wurde nicht explizit dargestellt, da er von dem Kompressionsdruck überlagert ist und daher nicht direkt auswertbar ist.

Zur Auswertung und Beurteilung der Qualität unterschiedlicher Kraftstoffe können nun verschiedene Algorithmen angewendet werden. Dabei kann einerseits die Lage der Kurven A,B in Bezug auf den Kurbelwellenwinkel ϕ und andererseits das Integral über die beiden Kurven A,B ausgewertet werden.

Eine erste Möglichkeit zur Auswertung der beiden Kurven A,B besteht darin, dass aus der Verschiebung der beiden ansteigenden Bereiche der Kurven A,B mit dem Druckanstieg einen Rückschluss auf den Zündverzug der geprüften Kraftstoffe zu bestimmen, da der Druckanstieg ein Maß für den Beginn der Verbrennung des eingespritzten Kraftstoffs ist. Ein geringer Zündverzug bedeutet, dass die Cetanzahl CZ groß ist. Dieses ist bei der Kurve A der Fall. Bei der Kurve B besteht ein Zündverzug von etwa 2° Kurbelwellenwinkel. Somit kann daraus geschlossen werden, dass die Cetanzahl des Kraftstoffs B etwa zwei Einheiten niedriger ist als beim Kraftstoff A. Die beiden Kurven A, B repräsentieren somit zwei Kraftstoffe mit unterschiedlicher Qualität.

Eine weitere alternative Möglichkeit zur Bestimmung der Qualität eines Kraftstoffs besteht darin, dass über die beiden Kurven A, B jeweils das Integral gebildet wird. Dieser Algorithmus liefert ein besonders zuverlässiges Ergebnis. Wird nun beispielsweise der Kraftstoff A mit einer vorgegebenen Menge und bei einem vorgegebenen Betriebszustand in einen Dieselmotor eingespritzt, dann entspricht das Integral über die Kurve A der gewonnenen Arbeit, die bei der Verbrennung als mechanisches Drehmoment der Brennkraftmaschine entnehmbar ist. Die Aufzeichnung der beiden Kurven A und B erfolgte unter gleichen Betriebsbedingungen der Brennkraftmaschinen, insbesondere identische Einspritzmengen wurden bei beiden Kurven verwendet. Das Integral über die Kurve B ist größer als das Integral über die Kurve A, so dass die Dichte bzw. der Heizwert des Kraftstoffs B größer als der des Kraftstoffs A ist. Das heißt, bei gleicher Einspritzmenge fällt mit abnehmender Dichte die der Brennkraftmaschine zugeführte Energie geringer aus, so dass die Brennkraftmaschine auch nur eine geringere Leistung abgeben kann. Damit aber die Anforderungen an die Abgasemissionen und insbesondere an die Partikelbildung erfüllt werden können, ist entsprechend der Erfindung eine Korrektur der Einspritzparameter, beispielsweise die einzuspritzende Kraftstoffmenge, der Einspritzdruck, der Einspritzzeitpunkt usw. vorgesehen. Diese Korrektur ist insbesondere dann notwendig, wenn im Kraftstofftank durch Auftanken Kraftstoffe mit unterschiedlicher Qualität miteinander vermischt werden. Es ist daher vorgesehen, insbesondere nach dem Auftanken eine Überprüfung der Kraftstoffqualität durchzuführen. Dieses Verfahren kann sowohl bei einem Dieselmotor als auch analog bei einem Benzinmotor angewendet werden.

In der Praxis kann das oben dargestellte Verhalten auf sehr einfache Weise mit einem Sensor ermittelt werden, der entweder an der Brennkraftmaschine oder im Abgassystem angeordnet ist. Eine bevorzugte, sehr einfache Ausführung der erfindungsgemäßen Vorrichtung wird mit einem Drucksensor gesehen, der den Druck im Brennraum der Brennkraftmaschine erfasst.

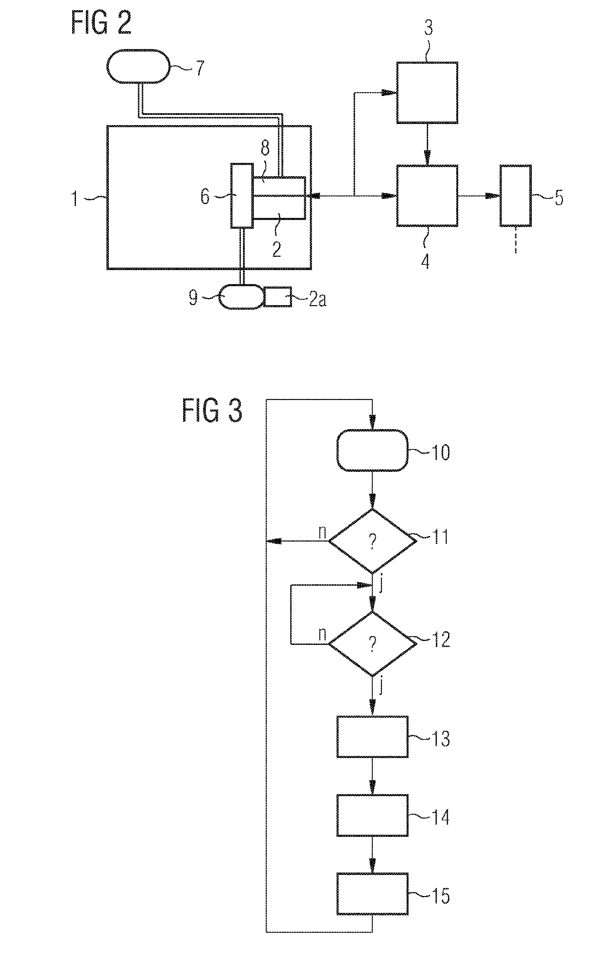

Figur 2 zeigt ein Ausführungsbeispiel, bei dem eine erfindungsgemäße Vorrichtung in Form eines Blockschaltbildes dargestellt ist. Figur 2 zeigt eine Brennkraftmaschine 1. Die Brennkraftmaschine 1 weist wenigstens einen Zylinder mit einem Brennraum 6 auf, an dem ein Sensor 2 angeordnet ist. Die Brennkraftmaschine 1 ist als Diesel- oder Benzinmotor mit einem Common-Rail-Einspritzsystem oder einem beliebigen Kraftstoffzuführsystem ausgebildet. Zur Kraftstoffzuführung wird für jeden Zylinder bzw. Brennraum 6 beispielsweise wenigstens ein Kraftstoffinjektor 8 verwendet, der von einem Motorsteuergerät 4 steuerbar ist.

Des Weiteren ist der Kraftstoffinjektor 8 mit einem Kraftstofftank 7 verbunden, in dem der zu beurteilende Kraftstoff eingefüllt ist.

Im ersten Ausführungsbeispiel der Erfindung wird ein Drucksensor 2 verwendet, der zur Aufnahme des Brennraumdruckes ausgebildet ist. Der Drucksensor 2 liefert einerseits seine Messwerte an das Motorsteuergerät 4 und andererseits an eine Vorrichtung 3 zur Auswertung dieser Messdaten. Die Vorrichtung 3 ermittelt mit dem vorgegebenen Algorithmus einen Faktor k, der ein Maß für die Qualität des momentan eingespritzten Kraftstoffs ist. Der Faktor k wird an das Motorsteuergerät 4 übertragen, so dass das Motorsteuergerät 4 neben den bereits erfassten und verarbeiteten Betriebsparametern, beispielsweise dem Einspritzdruck, dem Einspritzzeitpunkt, der Einspritzdauer, der Abgasrückführung usw. auch die einzuspritzende Kraftstoffmenge entsprechend dem Faktor k korrigieren kann. Der Faktor k kann dabei in einer beliebigen Funktion verarbeitet werden. Die Ermittlung der Qualität des Kraftstoffs erfolgt dabei bei einem vorgegebenen konstanten Betriebspunkt der Brennkraftmaschine 1. Dadurch ist es möglich, den Faktor k beispielsweise durch gespeicherte Werte für vergleichbare Kraftstoffe auf sehr einfache Weise aus einer Tabelle auszulesen. Als Algorithmus wird vorzugsweise das Integral über die gewonnene Kurve entsprechend dem Diagramm in Figur 1 ermittelt, die aus den Messwerten während der Verbrennung des Kraftstoffes gebildet wird. Die integrierte Kurve stellt somit ein Maß für die gewonnene Arbeit dar, die bei der vorgegebenen Einspritzmenge an Kraftstoff gewonnen werden kann. Je kleiner die gewonnene Arbeit ist, desto geringer ist die Dichte bzw. der Heizwert des Kraftstoffes. Das bedeutet, dass im Vergleich zu einem höherwertigeren Kraftstoff beispielsweise die einzuspritzende Kraftstoffmenge, die Abgasrückführrate usw. durch den Faktor k vergrößert werden muss.

Wie bereits erwähnt, kann alternativ und/oder auch der Zündverzug zur Beurteilung des eingespritzten Kraftstoffs verwendet werden, da beispielsweise ein geringer Zündverzug für einen Kraftstoff mit einer höheren Cetanzahl angesehen werden kann.

Eine weitere alternative Möglichkeit zur Beurteilung der Qualität eines Kraftstoffs kann auch durch eine Lambda-Sonde 2a gesehen werden. Die Lambda-Sonde 2a ist in einem Abgassystem 9 der Brennkraftmaschine eingebaut und erfasst den im Abgas befindlichen Restsauerstoff. Ähnlich, wie zuvor beschrieben, ergeben sich bei unterschiedlichen Kraftstoff-Qualitäten auch unterschiedliche Lambdawerte, wenn die Messungen bei jeweils gleichen Betriebsbedingungen der Brennkraftmaschine mit gleichen Einspritzparametern durchgeführt werden. Die Lambda-Sonde 2a hat ihre größte Genauigkeit, wenn der Lambdawert nahe bei dem Wert eins liegt. Erfindungsgemäß wird daher der Lambdawert bei Betriebsbedingungen gemessen, bei denen der Lambdawert nahe bei dem Wert eins liegt. Ein größerer Lambdawert, d.h. bei einem mageren Kraftstoff-Luftgemisch ist der restliche Sauerstoffanteil im Abgas niedriger. Analog ist im umgekehrten Fall der Sauerstoffanteil höher, wenn das Kraftstoff-Luftgemisch fett ist und somit der Lambdawert größer als eins ist.

Es ist vorgesehen, dieses Verfahren bei Diesel- als auch bei Benzinmotoren zu nutzen.

Figur 3 zeigt ein Flussdiagramm, mit dem die Beurteilung eines Kraftstoffs durchgeführt werden kann. In Position 10 beginnt das Programm. Der Motor wird gestartet und danach wird in Position 11 geprüft, ob eine neue Betankung des Kraftstofftanks erfolgt ist. Diese Überprüfung kann am einfachsten mit einer Abfrage der Füllstandsanzeige durchgeführt werden. Ist das nicht der Fall, springt das Programm bei n auf Position 10 zurück.

Wurde dagegen bei j der Kraftstofftank zumindest teilweise aufgefüllt, dann wird in Position 12 geprüft, ob ein vorgegebener konstanter Betriebspunkt der Brennkraftmaschine 1 erreicht ist. Ist das nicht der Fall, dann springt das Programm bei n wieder auf die Prüfroutine 12 zurück. Im anderen Fall erfolgt bei j in Position 13 eine Sensormessung, die beispielsweise mit dem Drucksensor 2 oder der Lambda-Sonde 2a durchgeführt werden kann. Gegebenenfalls kann die Messung mehrfach wiederholt werden und ein entsprechender Mittelwert gebildet werden, um ein zuverlässigeres Ergebnis zu erhalten. In Position 14 erfolgt die Auswertung mit dem vorgegebenen Algorithmus. Als Ergebnis wird ein Faktor k bestimmt, der in Position 15 gespeichert wird. Der gefundene Faktor k wird dann dem Motorsteuergerät 4 zugeführt, um eine entsprechende Anpassung für die einzuspritzende Kraftstoffmenge durchzuführen. Dabei können Parameter, wie der Einspritzdruck, der Einspritzzeitpunkt, die Einspritzdauer, die Abgasrückführung usw. angepasst werden. Beispielsweise kann bei einem zu geringen Heizwert die Einspritzzeit oder die Einspritzdauer verlängert werden. Auf diese Weise kann sehr einfach eine Optimierung der Kraftstoffzufuhr im Hinblick auf eine gewünschte Drehmomentenanforderung und/oder Einhaltung von Abgasemissionen durchgeführt werden.

## Patentansprüche

1. Verfahren zur Beurteilung der Qualität eines Kraftstoffs, insbesondere eines Diesel- oder Benzinkraftstoffs, wobei der Kraftstoff in einen Brennraum (6) einer Brennkraftmaschine (1) eingespritzt wird und wobei an der Brennkraftmaschine (1) wenigstens ein Sensor (2,2a) angeordnet ist, mit dem ein Betriebsparameter der Brennkraftmaschine (1), vorzugsweise ein Brennraumdruck und/oder ein Lambdawert erfasst wird, **dadurch gekennzeichnet, dass** der während der Verbrennung des Kraftstoffs im Brennraum (6) entstehende Druckverlauf und/oder während eines vorgegebenen, konstanten Betriebspunktes der Brennkraftmaschine (1) wenigstens ein entsprechender Lambdawert erfasst wird, dass der erfasste Druckverlauf und/oder der wenigstens eine Lambdawert mit einem vorgegebenen Algorithmus ausgewertet werden und dass als Ergebnis ein kraftstoffspezifischer Faktor k bestimmt wird, mit dem die Qualität des Kraftstoffs beurteilt wird.

2. Verfahren nach Anspruch 1, **dadurch gekennzeichnet, dass** der Faktor k einem Motorsteuergerät (4) zugeführt wird und dass das Motorsteuergerät (4) mit Hilfe des Faktors k die Betriebsparameter, beispielsweise den Kraftstoffdruck, die Abgasrückführrate, das Einspritzmuster, die einzuspritzende Kraftstoffmenge, den Einspritzbeginn, das Einspritzende für die Brennkraftmaschine korrigiert.

3. Verfahren nach Anspruch 1 oder 2, **dadurch gekennzeichnet, dass** zur Beurteilung der Kraftstoffqualität der Druckverlauf im Brennraum (6) während der Verbrennung innerhalb eines vorgegebenen Winkelbereiches der Kurbelwelle der Brennkraftmaschine (1) integriert wird.

4. Verfahren nach Anspruch 3, **dadurch gekennzeichnet, dass** aus dem integrierten Druckverlauf durch Vergleich mit gespeicherten oder errechneten Qualitätswerten von Kraftstoffen die Dichte beziehungsweise der Heizwert des eingespritzten Kraftstoffs bestimmt wird.

5. Verfahren nach Anspruch 1, **dadurch gekennzeichnet, dass** zur Beurteilung der Qualität des Kraftstoffs ein Zündverzug ermittelt wird, wobei der Zündverzug aus einem verzögerten Anstieg des Brennraumdrucks abgeleitet wird.

6. Verfahren nach Anspruch 1, **dadurch gekennzeichnet, dass** als Sensor eine Lambda-Sonde (2a) verwendet wird, dass bei einem vorgegebenen, konstanten Betriebspunkt der Brennkraftmaschine (1) ein aktueller Lambdawert gemessen wird, dass der aktuelle Lambdawert mit einem für diesen Betriebspunkt vorgegebenen, gespeicherten oder errechneten Lambda-Sollwert verglichen wird und dass die Differenz aus den beiden Lambdawerten als Maß für die Beurteilung der Qualität des Kraftstoffs verwendet wird.

7. Verfahren nach Anspruch 6, **dadurch gekennzeichnet, dass** der konstante Betriebspunkt der Brennkraftmaschine (1) derart gewählt wird, dass der Lambda-Sollwert nahe dem Wert eins liegt.

8. Verfahren nach einem der vorhergehenden Ansprüche, **dadurch gekennzeichnet, dass** die Beurteilung der Kraftstoffqualität wenigstens nach dem Auffüllen eines Kraftstofftanks (7) für die Brennkraftmaschine (1) durchgeführt wird.

9. Vorrichtung zur Beurteilung der Qualität eines Kraftstoffs, insbesondere eines Diesel- oder Benzinkraftstoffs für ein Verfahren nach einem der vorhergehenden Ansprüche, mit einem Brennraum (6) einer Brennkraftmaschine (1), mit wenigstens einem Sensor (2,2a) und mit einem Motorsteuergerät (4), **dadurch gekennzeichnet, dass** die Vorrichtung eine Auswerteeinheit (3) aufweist, dass die Auswerteeinheit (3) einen Algorithmus zur Auswertung der Sensorsignale aufweist und dass die Vorrichtung ausgebildet ist, aus den Sensorsignalen die Qualität des verwendeten Kraftstoffs zu beurteilen.

10. Vorrichtung nach Anspruch 9, **dadurch gekennzeichnet, dass** der Sensor als Drucksensor (2) ausgebildet ist und den Brennraumdruck während der Verbrennung des Kraftstoffs erfasst.

11. Vorrichtung nach Anspruch 9, **dadurch gekennzeichnet, dass** der Sensor als Lambdasonde (2a) ausgebildet ist und dass die Lambdasonde (2a) im Abgassystem (9) der Brennkraftmaschine (1) angeordnet ist.
